# EUROPEAN PATENT SPECIFICATION

(11) **EP 1 608 368 B1**
(45) Date of publication and mention of the grant of the patent: **01.10.2008**
(21) Application number: 04720871.5
(22) Date of filing: 16.03.2004
(51) Int. Cl.: A61K 31/436, A61K 31/445, A61P 37/00

(54) **PHARMACEUTICAL COMPOSITIONS COMPRISING A COMBINATION OF RAPAMYCIN OR ITS DERIVATIVE AND PIMECROLIMUS FOR THE TREATMENT OF INFLAMMATORY BOWEL DISEASE (IBD)**
PHARMAZEUTISCHE ZUSAMMENSETZUNGEN MIT EINER KOMBINATION AUS RAPAMYCIN ODER SEINEN DERIVATEN UND PIMECROLIMUS ZUR BEHANDLUNG VON ENTZÜNDLICHER DARMERKRANKUNG (IBD)
COMPOSITIONS PHARMACEUTIQUES COMPRENANT UNE COMBINAISON DE RAPAMYCINE OU DE SON DERIVE ET DE PIMECROLIMUS POUR LE TRAITEMENT DE L' AFFECTION INTESTINALE INFLAMMATOIRE (IBD)

(30) Priority: 17.03.2003 GB 0306070; 25.03.2003 GB 0306868; 15.08.2003 GB 0319226
(43) Date of publication of application: 28.12.2005
(73) Proprietor: Novartis AG, 4056 Basel (CH); Novartis Pharma GmbH, 1230 Wien (AT)
(72) Inventor: MOORE, Henrietta, A-2384 Breitenfurt (AT)
(86) International application number: PCT/EP2004/002714
(87) International publication number: WO 2004/082681

(56) References cited:
- EP-A- 1 208 847
- WO-A-99/24036
- WO-A-02/089796
- WO-A-03/057218
- WO-A-03/094990
- US-A- 6 004 973
- MARSLAND ALEXANDER M ET AL: "The macrolide immunosuppressants in dermatology: mechanisms of action." [Online] November 2002 (2002-11), EUROPEAN JOURNAL OF DERMATOLOGY : EJD. 2002 NOV-DEC, VOL. 12, NR. 6, PAGE(S) 618 - 622 , XP002284186 ISSN: 1167-1122 John Libbey Eurotext Retrieved from the Internet: URL:http://www.john-libbey-eurotext.fr/en/ revues/medecine/ejd/e-docs/00/01/87/58/art icle.md?type=text.html> [retrieved on 2004-06-07] page 2, paragraph 5
- BORNHÖVD E ET AL: "Macrolactam immunomodulators for topical treatment of inflammatory skin diseases." November 2001 (2001-11), JOURNAL OF THE AMERICAN ACADEMY OF DERMATOLOGY. NOV 2001, VOL. 45, NR. 5, PAGE(S) 736 - 743 , XP002284187 ISSN: 0190-9622 the whole document

## Description

The present invention relates to the use of pharmaceutical compositions for the treatment of Inflammatory Bowel Disease (IBD) and diseases associated therewith.

In one aspect the present invention provides the use of a combination of rapamycin or a rapamydn derivative and a compound of formula 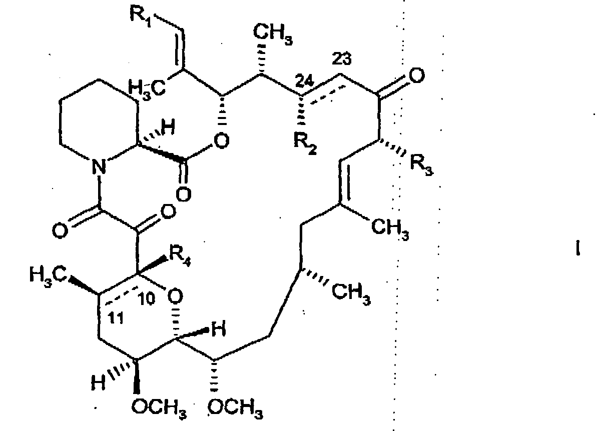 wherein either
R₁ is a group (a) of formula 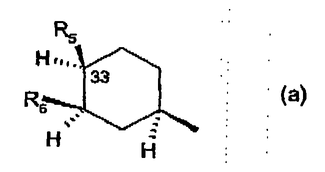 wherein
R₅ is chloro, bromo, iodo or azido,
R₆ is hydroxy or methoxy, and
R₄ is hydroxy and there is a single bond in 10,11 position; or absent, and there is a double bond in 10,11 position
or
R₁ is a group (b) or (c) of formula 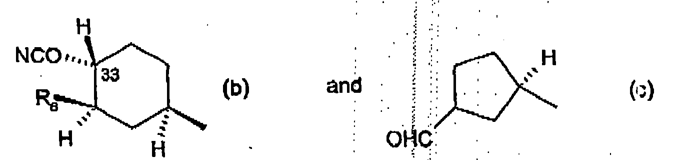 wherein
R₆ is as defined above, and
R₄ is hydroxy and there is a single bond in 10,11 position,
R₂ is oxo and there is a single bond in 23,24 position; optionally protected hydroxy and there is a single or double bond in 23,24 position; or absent and there is a double bond in 23,24 position;
R₃ is methyl, ethyl, propyl or allyl
beside at least one pharmaceutically acceptable excipient, for the manufacture of a medicament for the treatment of Inflammatory Bowel Disease and diseases associated therewirth.

IBD in man, like many other chronic inflammatory or autoimmune diseases, can be seen as a result of a convergence of interactions between susceptibility genes, the environment and the immune system. The extent of interplay between these factors will deterrnine the clinical manifestation of disease; Crohn's disease (CD) or ulcerative colitis (UC) and the various clinical subgroups as defined by severity and location. The leading current hypothesis on the pathogenesis of IBD is that it is a dysregulated immune response to normal enteric bacterial antigens (see e.g. Kirsner JB, Inflammatory Bowel Disease, 5th ed; W.B. Saunders, p. 208-39, p. 299-304; p. 305-14 and p. 315-25). In both CD and UC the number of lamina propria T lymphocytes is increased (see e.g. Selby et al, (1984), Gut; 25 (1):32-40). There is evidence, mostly from experimental models such as the SCID-IBD model, that suppressor subsets of T cells prevent a reaction to the normal flora encountered in the gut (see e.g. Groux H, et al, (1999), Immunol. Today; 20 (10):442-5). In IBD this balance has been disturbed and the ensuing inflammatory cascade develops into a chronic response. The transfer of syngeneic CD4⁺ T cells which express high levels of CD45RB (DD4⁺CD45RB^{Hi}) to SCID mice (SCID-IBD) induces a severe colitis and wasting disease which usually results in death of the mice within 4-8 weeks following transfer of the pathological CD4⁺CD45RB^{Hi} T cells (see e.g. Powrie F et al (1993), Int.Immunol.; 5 (11):1461-71). This severe model of colitis shares many of the features of human IBD and has indeed been used throughout the 1990's to enhance further understanding of the disease in man and is regarded to reflect IBD more than any other model. These include colitis induced by administration of exogenous agents or by gene manipulation (see e.g. Blumberg RS et al, (1999) Curr.Opin.lmrnunol.; 11 (6):648-56; Strober W et al, (1998), Ann.Intern.Med.; 128 (10):848-56; Strober W et al, (1998), Scand.J.Immunol.; 48 (5):453-8). Using the SCID-IBD model, antibodies against TNFα, IFNγ and IL-12 have shown transient and partial beneficial effects (see e.g. Powrie F et al (1994), Immunity.; 1 (7):553-62; Simpson SJ, et al (1998), J.Exp.Med.; 187 (8):1225-34; Mackay F et al (1998), Gastroenterology; 115 (6):1464-75). More recently, beneficial effects have also been reported with antibodies to the CD134 ligand (to OX40L, see e.g. Malmstrom V et al (2001), J.Immunol.; 166 (11):6972-81); to CD154 (see e.g. Liu Z. et al, (2000), J.Immunol.; 164 (11):6005-14; De Jong YP et al (2000), Gastroenterology; 119 (3):715-23) and to the β7 integrin or MAdCAM-1 (see e.g. Picarella D et al, (1997), J.Immunol.; 158 (5):2099-106). Together these data demonstrate that the disease can be abrogated by inhibition of either the main inflammatory mediators including TNFα, and/or cytokines involved in neutralizing Th1 type differentiation and effector function or by interfering with the antigen presentation steps occurring in the lymph nodes or with the process of leucocyte homing of these cells.

A pharmaceutical composition comprising in combination rapamycin or a rapamycin derivative and a compound of formula I is hereinafter designated as "a composition of (according to) the present invention".

In another aspect of the present invention the compound of formula I is a compound of formula

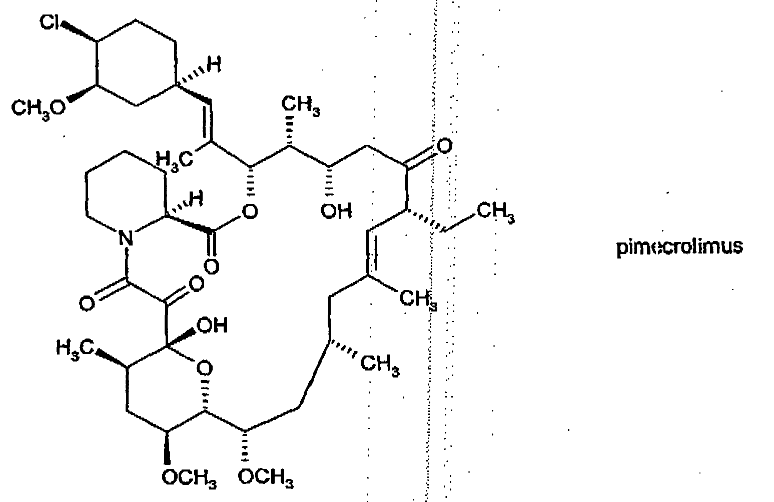

Pimecrolimus (INN recommended) (ASM981; Elidel^{™}), i.e.

{[1E-(1R,3R,4S)]1R,9S,12S,13R,14S,17R,18E,21S,23S,24R,25S,27R}-12-[2-(4-chloro-3-methoxycyclohexyl)-1-methylvinyl]-17-ethyl-1,14-dihydroxy-23,25-dimethoxy-13,19,21,27-tetramethyl-11,28,dioxa-4-azatricyclo [22.3.1.0(4,9)]octacos-18-ene-2,3,10,16-tetraone is disclosed e.g. in EP 427 680 (33-epi-33-chloro-FR 520 of example 66a).

In another aspect of the present invention the rapamycin derivative is a compound of formula 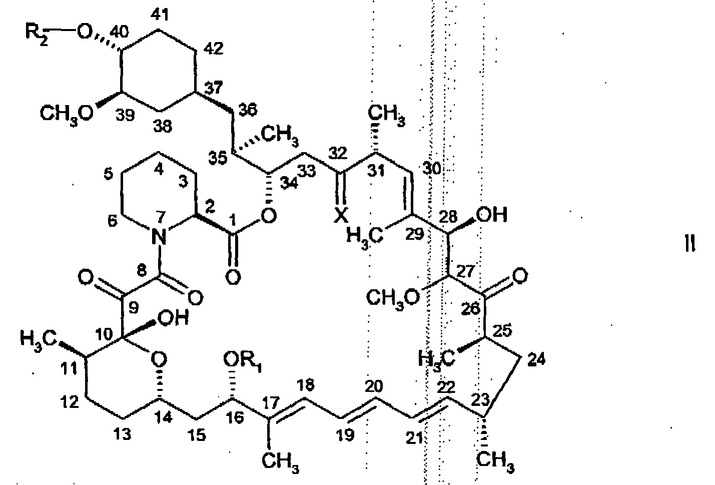 wherein
R₁ is CH₃ or (C₃₋₆)alkynyl,
R₂ is H, -CH₂-CH₂-OH, 3-hydroxy-2-(hydroxymethyl)-2-methyl-propanoyl or tetrazolyl, and
X is =O, (H,H) or (H,OH),
provided that R₂ is other than H when X is =O and R₁ is CH₃,
or a prodrug thereof when R₂ is -CH₂CH₂-OH, e.g. a physiologically hydrolysable ether thereof.

Representative rapamycin derivatives of formula II include e.g. 32-hydrogenated rapamycin e.g. as described in WO 96/41807 and US 5 256 790, incorporated herein by reference, such as e.g. 32-deoxorapamycin, 16-pent-2-ynyloxy-32-deoxorapamycin, 16-O-substituted rapamycin e.g. as disclosed in WO 94/02136, WO 95/16691 and WO 96/41807, the contents of which are incorporated herein by reference, such as e.g. 16-pent-2-ynyloxy-32(S or R)-dihydro-rapamycin, 16-pent-2-ynyloxy-32(S or R)-dihydro-40-O-(2-hydroxyethyl)-rapamycin, 40-O-substituted rapamycin e.g. as described in US 5 258 389, WO 94/09010, WO 92/05179, US 5 118 677, US 5 118 678, US 5 100 883, US 5 151 413, US 5 120 842, WO 93/11130, WO 94/02136, WO 94/02485 and WO 95/14023, all of which are incorporated herein by reference, such as e.g. 40-[3-hydroxy-2-(hydroxymethyl)-2-methylpropanoate]-rapamycin (also designated as CCI779) or 40-epi-(tetrazolyl)-rapamycin (also designated ABT578). A preferred compound is e.g. 40-0-(2-hydroxyethyl)-rapamycin (Compound A hereinafter), e.g. disclosed in Example 8 in WO94/09010, or 32-deoxorapamycin, or 16-pent-2-ynyloxy-32(S)-dihydro-rapamycin, e.g. disclosed in WO96/41807. Rapamycin derivatives may also include (epi)rapalogs, e.g. as disclosed in WO98/02441 and WO01/14387, e.g. the compounds AP23573, AP23464, AP23675 or AP23841; or derivatives such as biolimus 7 or biolimus 9. Rapamycin derivatives may also include prodrugs of rapamycin such as TAFA93.
Suitable rapamycin derivatives are e.g. also described in US 3 929 992 and US 5 258 389. Preferably the compositions of the present invention comprise rapamycin, also known as sirolimus (rapamycin; Rapamune^{R}) and/or the rapamycin derivative everolirnus (Compound A, RAD001; 40-O-(2-hydroxyethyl)-rapamycin; Certican^{R}).
All patent literature cited herein is introduced herein by reference.

In another aspect the present invention the pharmaceutical composition comprising in combination 40-O-(2-hydroxyethyl)-rapamycin and pimecrolimus comprises at least one pharmaceutically acceptable excipient, e.g. appropriate carrier and/or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or bluffers.

A compound of a combination according to the present invention, i.e. a compound of formula I, e.g. pimecrolimus, may be in free form, in the form of a salt, in solvate form or in the form of a salt and a solvate, where salts and/or solvates exist.

In another aspect of the present invention the compound of formula I is in the form of a salt.

A composition of the present invention may comprise one or more rapamycin derivatives, preferably rapamycin or one rapamycin derivative, and one or more compounds of formula I, preferably one, e.g. pimecrolimus.

A composition of the present invention is suitably based on emulsions, microemulsions, emulsion preconcentrates or microemulsion preconcentrates, or solid dispersions, especially water-in-oil microemulsion preconcentrates or oil-in-water microemulsions of rapamycin or a rapamycin derivative, e.g. a compound of formula II, and a compound of formula I, e.g. pimecrolimus.

A composition of the present invention may further comprise further pharmaceutically active compounds. Such further active compounds e.g. include anti-inflammatory and immunomodulatory agents.

In another aspect the pharmaceutical composition of the present invention comprises further an anti-inflammatory and/or an immunomodulatory agent.

A composition of the present invention includes
- fixed combinations, in which a compound of formula I, e.g. pimecrolimus, and rapamycin or a rapamycin derivative, e.g. a compound of formula II, such as Compound A, are in the same formulation;
- kits (=kit of parts), in which a compound of formula I, e.g. pimecrolimus, and rapamycin or a rapamycin derivative, e.g. a compound of formula II, such as Compound A, in separate formulations are sold in the same package, e.g. with instruction for co-administration; and
- free combinations in which a compound of formula I, e.g. pimecrolimus, and rapamycin or a rapamycin derivative, e.g. a compound of formula II, such as Compound A, are packaged separately, but instruction for simultaneous or sequential administration are given.

In another aspect the present invention provides a pharmaceutical kit of parts comprising rapamycin or a rapamycin derivative, e.g. a rapamycin derivative of formula II, e.g. Compound A, and a compound of formula I, e.g. pimecrolimus, beside instructions for simultaneous or sequential administration, for the manufacture of a medicament for the treatment of IBD and diseases associated therewith.

We also have found surprisingly a pharmaceutical composition, e.g. solid, such as a tablet, comprising a fixed combination of rapamycin or a rapamycin derivative, including a compound of formula II, e.g. Compound A, and a compound of formula I, e.g. pimecrolimus, as active agents, e.g. an oral pharmaceutical composition.

In another aspect the present invention provides a pharmaceutical composition, e.g. an oral pharmaceutical composition as a fixed combination, e.g. a solid oral composition, in the form of a solid dispersion comprising rapamycin or a rapamycin derivative, e.g. Compound A, and a compound of formula I, e.g. pimecrolimus, and a carrier, e.g. cellulose, optionally in the presence of excipients as mentioned above:

Compositions in the form of a solid dispersion may be administered in any convenient form, e.g. tablet, capsule, granule or powder form.

Pharmaceutical compositions of the present invention, including an oral pharmaceutical composition, may be prepared as appropriate, e.g. according, such as analogously, to a method as conventional, or as described herein.

For the preparation of a fixed oral composition or combination, e.g. a tablet, of the present invention, a solid dispersion comprising a compound of formula I, e.g. pimecrolimus, and rapamycin or a rapamycin derivative, e.g. a compound of formula II, and a carrier, such as a cellulose, e.g. hydroxypropylmethylcellulose, may be used. Solid dispersions are known or may be obtained as appropriate. A tablet may be obtained e.g. by mixing the active agents with a carrier and optionally further excipient, dry granulation before or after adding further excipient, and compression of the mixture obtained. Further excipient includes e.g.
- lubricants, such as Mg-stearate,
- fillers, e.g. sugars, such as lactose,
- flowing agents, such as siliciumdioxide, e.g. aerosils,
- disintegrants, such as polyvinylpyrrollidones, e.g. povidones, crospovidones (homo- or copolymers of N-vinyl-pyrrolidone).

The weight ratio of a compound of formula I, e.g. pimecrolimus, and rapamycin or a rapamycin derivative, e.g. a compound of formula II, in an oral composition of the present invention is not critical, e.g. including a weight ratio of from, e.g. about, 1:10 to 800:1. If a compound pimecrolimus and Compound A are used as active agents, pimecrolimus is preferably used in an excess, e.g. an appropriate weight ratio of pimecrolimus : Compound A includes a ratio of from, e.g. about, 10:1 to 800:1.

In another aspect the present invention provides a tablet for oral administration, comprising
- Compound A and pimecrolimus as active agents, e.g. in the form of a solid dispersion,
- a cellulose as a carrier, e.g. hydroxpropyfmethylcellulose,
- optionally disintegrants, e.g. a polyvinylpyrrolidone,
- optionally flowing agents, e.g. an aerosil,
- optionally lubricants, e.g. Mg-stearate,
- optionally fillers, e.g. sugars such as lactose, for the manufacture of a medicament for the theatment of IBD and diseases associated therewith.

In another aspect the present invention provides a tablet of the present invention comprising in % by weight
- pimecrolimus: 2 to 8%, e.g. 4%;
- Compound A: 0.05 to 0.25%, e.g. 0.13%;
- hydroxypropylmethylcellulose (e.g. 3 cps): 10 to 30%, e.g. 21.4%;
- lactose (e.g. 200 mesh): 0.6 to 2.0%, e.g. 1.2%;
- butylated hydroxytoluene: 0.005 to 0.025%, e.g. 0.013%
- lactose spray dried: 25.0 to 70.0%, e.g. 50.8%;
- crospovidone: 10.0 to 40.0%, e.g. 20.0%;
- silicium dioxide colloidal (Aerosil 200®): 0.5 to 2.5%; e.g. 1.5%
- magnesium stearate: 0.5 to 2.0%, e.g. 1.0 %.

Such tablet may e.g. contain, e.g. about, 10 to 60 mg of pimecrolimus, e.g. 30 mg, and 0.1 to 2 mg of Compound A, e.g. 1 mg.

A composition of the present invention may be administered by any appropriate route, e.g. analogously to administration of a compound of formula I, e.g. pimecrolimus and rapamycin or a rapamycin derivative, e.g. a compound of formula II.

The compositions of the present invention may be prepared as appropriate, e.g. analogously to a method as conventional, e.g. by mixing a compound of formula I, e.g. pimecrolimus, and rapamycin or a rapamycin derivative, in combination or each separately, beside at least one pharmaceutically acceptable excipient.

We also have found that a compound class including FK506 in combination with rapamycin or a rapamycin derivative is useful in the treatment of inflammatory and immunologically-mediated diseases.

Utility of rapamycin derivatives of formula II and of combinations of the invention as described above in treating diseases and conditions as hereinabove specified, may be demonstrated in standard animal or clinical tests, e.g. as described hereinafter.

Daily dosages required in practicing the methods of the present invention will vary depending upon, for example, the chemical nature and the pharmacokinetic data of a compound of the present invention employed, the individual host, the mode of administration and the nature and severity of the conditions being treated. However, in general, for satisfactory results in larger mammals, for example humans, an indicated daily dosage is in a similar, but lower range, than the range in which a compound of formula I, e.g. pimecrolimus, and rapamycin or a rapamycin derivative are generally administered.
A preferred daily dosage range is about from 0.1 to 25 mg of rapamycin or a rapamycin derivative of formula II, e.g. Compound A, as a single dose or in divided doses. Suitable daily dosages for patients are on the order of from e.g. 0.1 to 25 mg p.o. of rapamycin or a rapamycin derivative of formula II, e.g. Compound A.
A composition of the present invention may be administered by any appropriate route, e.g. analogously to administration of a compound of formula I, e.g. pimecrolimus and rapamycin or a rapamycin derivative, e.g. a compound of formula II. For example, rapamycin or a rapamycin derivative of formula II, e.g. Compound A, may be administered by any conventional route, in particular enterally, e.g. orally, e.g. in the form of tablets, capsules, drink solutions, nasally, pulmonary (by inhalation) or parenterally, e.g. in the form of injectable solutions or suspensions. Suitable unit dosage forms for oral administration comprise from ca. 0.05 to 12.5 mg, usually 0.25 to 10 mg of rapamycin or a rapamycin derivative of formula II, e.g. Compound A, together with one or more pharmaceutically acceptable diluents or carriers therefore.

In the present description the terms "treatment" on "treat" refer to both prophylactic or preventive treatment as well as curative or disease modifying treatment, including treatment of patients at risk of contracting disease or suspected to have contracted disease as well as patients who are ill or have been diagnosed as suffering from a disease or medical condition.

### Description of the Figures

Figures 1 to Figure 3
   Show the body weight of the SCID-IBD mice after having been reconstituted with 2x10⁵ CD₄⁺CD45RB^{Hi} T cells, treated and untreated with pimecrolimus (ASM), Compound A and a combination of ASM + Compound A. PBS-mice are non-transferred mice. Treatment started on day 1 after transfer and continued daily. Data are expressed as the mean % body weight (relative to weight on day 0) ± s.e.m. for n=8 (with few exceptions where n=5, 6 or 7) mice per group **p<0.01, *p<0.05 relative to the vehicle-treated group the loss of body weight is shown.
   In Figure 1 the results from using a Dosage 1 (Compound A: 0.1 mg/kg/d, ASM: 60mg/kg/d), in Figure 2 the results from using a Dosage 2 (Compound A: 0.1 mg/kg/d, ASM: 30mg/kg/d, and
   in Figure 3 the results from using a Dosage 3 (Compound A: 0.05 mg/kg/d, ASM: 10 mg/kg/d) are shown.
Figure 4
   shows the severity of colon inflammation in SCIB-IBD mice after various treatments, severity scores are given as described in example 1.

Temperatures are given in degree Celsius (°) and are uncorrected
The following abbreviations are used herein:
- ASM: pimecrolimus
- AUC: Area under the Curve
- ANOVA: Analysis of variance
- BW: Body Weight
- CD: Crohn's disease
- CyA: Cyclosporin A
- FACS: Fluorescence Activated Cell Sorter
- H & E: Hæmatoxylin & eosin
- HTAB: Hexadecyltrimethylammoniumbromide
- IBD: Inflammatory Bowel Disease
- IFN: Interferon
- IL-: Interleukin
- i.p.: Intra-peritoneal
- MPO: Myeloperoxidase
- MLN: Mesenteric lymph node
- MTOR: mammalian Target of Rapamycin
- PBS: Phosphate buffered saline
- PBS-(mice): SCID mice receiving PBS i.p. instead of disease-causing T cells
- p.o.: Per os
- RAD: Compound A, everolimus
- SCID: Severe Combined ImmunoDeficient
- s.e.m.: Standard error of the mean
- SD: Standard deviation
- TMB: 3,3',5,5'-Tetramethylbenzidine
- TNF: Tumour necrosis factor
- UC: Ulcerative colitis

### Example 1

### Test methods

### SCID-IBD Mouse Model

Female BALB/cJ and C.B.17 scid/scid mice (Fox Chase SCID^{®}, Bomholtgaard, Denmark) are maintained in ventilated cage racks (Micro-vent type 84-II, Allentown Caging Equipment Co., Allentown, New Jersey, USA) under specific pathogen free conditions. The experimental procedures performed on the mice are authorized under the license number MA 1048/00 (Magistratabteilung No. 58, Amt der Wiener Landesregierung).
Briefly, CD4⁺CD45RB^{Hi} T lymphocytes are isolated from BALB/c mouse spleens by two-colour FACS-sorting through a live/dead gate and injected (2 x 10⁵ cells/mouse, i.p.) into 6-9 week old female SCID mice. Negative control mice receive PBS i.p. and one such mouse is in each cage as a sentinel to monitor possible infections in this immunodeficient colony (PBS-mice).
SCID mice are first reconstituted with the CD4⁺C1745RB^{Hi} T cells and then treated with test compounds or vehicle as controls. As test compounds Compound A (RAD) and pimecrolimus (ASM) are used. 3 separate studies using different dosaging are carried out. In each study both compounds are administered alone and combine. All 3 studies follow the same basic protocol of 4 groups of mice with one group receiving, by daily oral gavage, both vehicles (placebo), one group Compound A alone, one group ASM alone, and one group a combination of Compound A and pimecrolimus, from day 1 to day 29 after cell transfer. Dosages administered:

| | |
|---|---|
| Study 1: Dosage 1: | Compound A: 0.1 mg/kg/d, ASM: 60mg/kg/d |
| Study 2: Dosage 2: | Compound A: 0.1 mg/kg/d, ASM: 30mg/kg/d, and |
| Study 3: Dosage 3: | Compound A: 0.05 mg/kg/d, ASM: 10 mg/kg/d. |

The body weight of each mouse is monitored throughout and at the end of the study mice are weighed.
The mice are anaesthetized at the end of the study and blood withdrawn via cardiac puncture for separation into serum which is frozen and stored at -80°.
Blood is also taken into 10 mM EDTA for preparation for FACS analysis.
The spleen and MLN are taken from each mouse, weighed and processed for FACS analysis of single cell suspensions.
A photographic picture is taken of the opened abdominal cavity and then a 1cm long piece of the colon, just above the rectum, is fixed in formalin for histology.
Specimens are embedded in paraffin, sections (3 µm thick) are cut and slained with H & E.

Four colour FACS analysis is performed on cell suspensions prepared from he blood, spleen and mesenteric lymph nodes (MLNs) of each mouse.
Antibodies are obtained from Pharmingen. Cells which are double positive for CD3 and CD4 are counted as lymphocytes. Cells positive for Ly-6G (myeloid differentiation antigen) and negative for CD3 or CD4 are neutrophils (also their cellular characteristics (forward and side scatter) distinguishes them from the other cells).
The number of cells per sample is calculated from the total cell number in each cell suspension and the % of positively identified (samples analysed on a FACS Calibur^{®} using CeliQuest Pro® software) cells per sample.
The mean of these values for a group of mice can thus be calculated. To process some of the data further, the determined values are adjusted by the background value found in non-transferred mice and calculating the % reduction, or indeed increase, in mean cell number, relative to the placebo treated group.
All data presented as mean values are qualified by an s.e.m. value. The body weight data are analysed either by repeated measures analysis or by first finding the mean AUC and then applying an ANOVA multiple comparison test with Tukey *post hoc* correction. Histology scores are represented as the mean severity score and compared with a Wilcoxon-Mann-Whitney test with exact p-values (StatXact-5 software) and Bonferroni *post hoc* correction factor. All other data are first analysed for normal distribution and then the ANOVA test for multiple comparison is applied. Statistical significance is taken as p<0.05.

The developing reaction in the SCID mouse to the transfer of CD4⁺CD45RB^{Hi} T cells shows that T cell expansion is the first event and the inflammatory sequelae follow, which can be measured as a critical loss of body weight, a severe colon inflammation and systemic signs such as increased serum haptoglobin levels and neutrophilia. The increasing T cell population with time also results in an increase in size of lymphoid organs which are otherwise comparatively small in the non-transferred SCID mouse. By day 28 post T cell transfer the inflammation is relatively consistent within a group of mice and this has been adopted as the shortest time where accurate effects of treatment with test compounds can be measured. The effects of a range of doses of Compound A and ASM as single preventative compounds are, in brief, comparative effective dose ranges are found to be from 30 to 100 mg/kg/d administration of ASM and 0.1 to 1mg /kg/d of Compound A. In the present tests sub-maximal doses of each compounds are used and the effect of the single and combined treatment in the same study is determined.

### Results

### Synergistic effect of combined treatment of SCID-IBD mice with Compound A and ASM on the loss of body weight

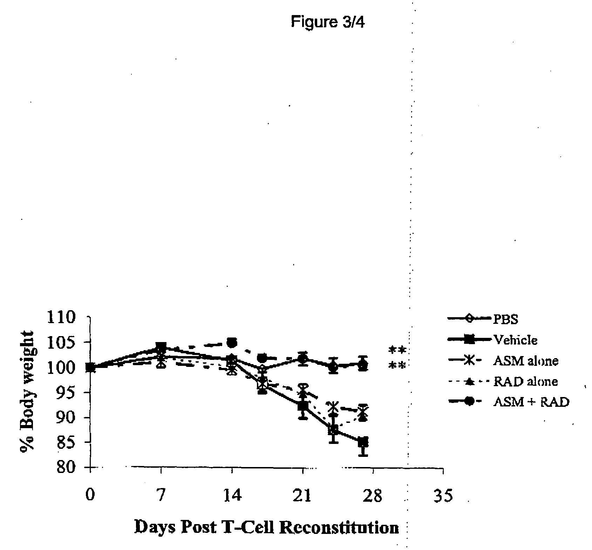

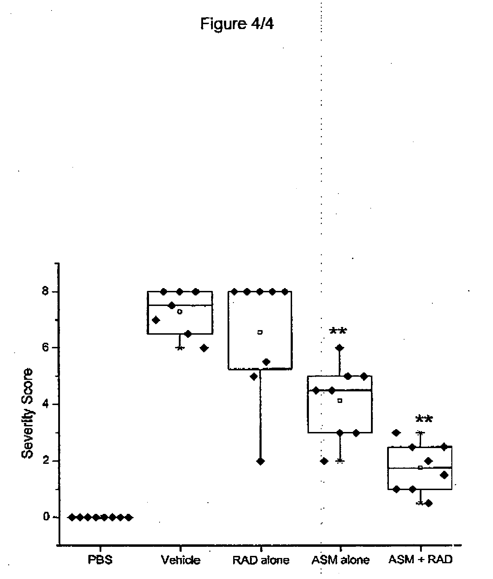

The first visible symptom resulting from the transfer of disease causing CD4⁺CD45RB^{Hi} T cells to SCID mice is a severe loss of body weight, usually significant 21 days after transfer. These mice (designated as placebo in these studies, e.g. in the Figures) continue to lose body weight until death occurs, generally 4 to 8 weeks after cell transfer. Non-transferred mice (designated as "PBS(-mouse)" in the Figures) remain stable or gain weight in the course of the study. In the 3 individual studies described herein, the mean % difference in body weight between the non-transferred and the vehicle-treated mice is calculated to be 16.3 ± 1.8%.
If transferred SCID mice are treated with Compound A alone or with ASM alone, a significant, but low inhibition of weight loss is only found for mice treated with ASM at a dosage of 60 mg/kg/d. Whereas thus dosages used of either compound alone have only minor effects on the loss of body weight, a combined administration of Compound A and ASM has a significant protective effect, even at the lowest doses of ASM (10 mg/kg/d) and Compound A (0.05 mg/kg/d), see e.g. Figure 3. Since the dosage administered of Compound A and ASM alone have no or only a small significant effect on weight loss, the protective effect of a combined administration cannot be additive, but is synergistic.

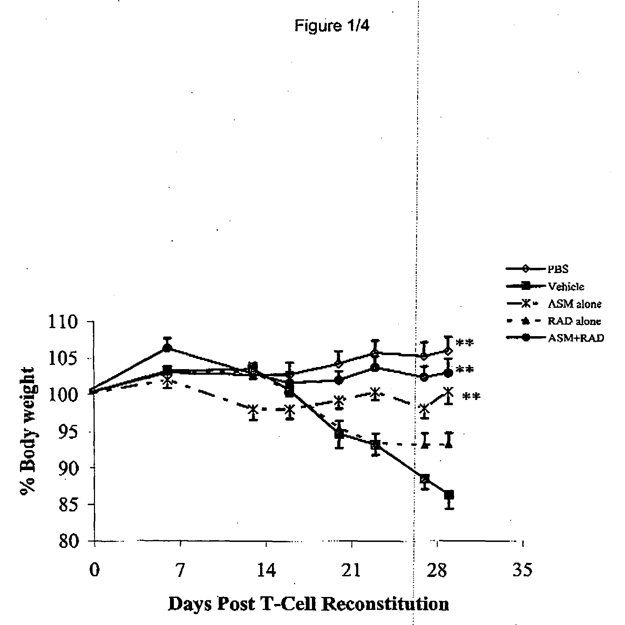

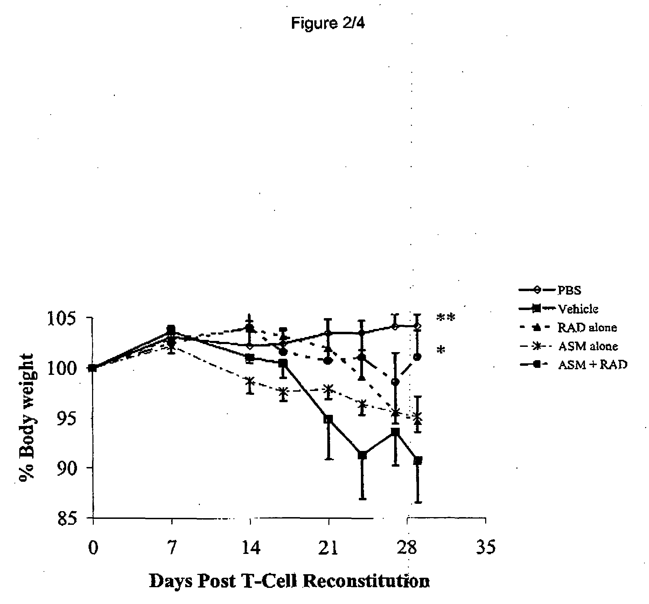

Further data (not shown) has been obtained which confirms the synergistic effect of a combination according to the present invention.
In short, we have found a synergistic effect of a combination of ASM and (compound A in the SCID-IBD model, when using sub-maximal doses of 60, 30 and 10 mg/kg/d of ASM and 0.1, 0.1 and 0.05 mg/kg/d of Compound A, respectively. The dose of 60 mg/kg/d of ASM alone still elicited significant effects on its own but all other single treatment groups resulted in only mild disease inhibition.
Specifically we have obtained the following results:
- All three combinations, most strikingly also the lowest doses of 10 mg/kg/d of ASM and 0.05mg/kg/d of Compound A, results in no loss of body weight (data shown, see Figures 1 to 3)
- At the low dose combinations of 10 and 0.05 mg/kg/d a synergistic effect on the colon inflammation is shown. Either compound alone had very little effect, whilst the severity scores in the combined treatment group are mostly mild (data not shown).
- ASM potently reduces serum haptoglobin levels at 60 and 30mg/kg/d whilst Compound A is very weakly active at 0.1 or 0.05 mg/kg/d. Again, synergism is evident in the low dose study where the % reduction of each compound alone was 32.7% (ASM 10 mg/kg/d) or 27.4% (Compound A, 0.05mg/kg/d) whereas the combined treatment group is significantly inhibited by 81.0% (data not shown).
- In the low dose study there is no significant reduction in lymphocyte numbers in any of the treatment groups, although the numbers in the combined group are reduced (46.5, 19.0 and 48.0% in blood, spleen and MLN, respectively) relative to vehicle-treated mice. In the study where ASM and Compound A are given at higher doses (30 mg/kg/d and 0.1 mg/kg/d, respectively; study 2) there are significant reductions in lymphocyte numbers (73.0, 80.3 and 90.7% in blood, spleen and MLN, respectively) whereas numbers in the single treatment groups (ASM or Compound A, respectively, administered alone) are not reduced. Similarly, with the higher dose of ASM (60 mg/kg/d; study 1), which alone decrease lymphocyte numbers specifically in the MLN (73.5%), the combination with Compound A (0.1 mg/kg/d) results in significant reductions in both blood (65.4%) and spleen (64.8%) in addition to an enhanced reduction (93.2%) in the MLN. The dose of 0.1 mg/kg/d of Compound A alone has only weak, if any effects on lymphocyte numbers.

### ASM981 combined with Compound A acts synergistically to reduce colon inflammation in SCID-IBD mice

The inflammation in the colon of SCID-IBD mice shares many features observed in lesional tissue of a Crohn's diseased colon. In order to examine the intestinal mucosal damage in SCID-IBD mice a histological examination of the colon sections was performed according to defined morphological parameters. The severe colitis associated with this model of IBD is characterized with a massive cellular infiltrate which in places involves nearly all of the submucosa. The infiltrate is mixed and comprised of lymphocytes, macrophages, neutrophils and some eosinophils. Also evident is the loss of crypt architecture, extensive elongation and epithelial hyperplasia. A severely inflamed colon section will also have crypt abscesses. Numeric scores were given as per the following scheme: a score of 0 to 3 is given for the leucocyte infiltrate where 1 is mild, 2 is moderate and 3 is a severe infiltrate including the submucosal layer. A score of 0 to 2 is given for Goblet cell depletion where 2 is given for a complete lack of the alcian blue staining, 0 is given for a normal amount of staining and 1 is intermediate. A score of 0 to 3 is given for mucosal hyperplasia and degenerative changes which include crypt elongation, epithelial hyperplasia, presence of crypt abscesses, erosions and epithelial necrosis. The maximum possible score is 8 and (PBS)-mice are scored 0.
We find that the dose of 60mg/kg/d ASM981 alone has a significant effect. Doses of Compound A below 0.5mg/kg/d are not significantly active and also not in this model. However, the two compounds together reduce the inflammation (Figure 5) to mild levels (scores below 4) that could not be achieved by the additive effects of each alone.
In conclusion, in the SCID-IBD model, at dosages which alone have minimal effects, combined treatment with pimecrolimus and Compound A results in highly significant prevention of the disease. The strong effect we see on body weight and colon inflammation does not correlate with the lymphocyte numbers alone. The synergistic effect is not exclusive to lymphocyte numbers but may be affecting lymphocyte activity or function as well as other cells involved in the disease. These data confirm that a combination of pimecrolimus and Compound A act synergistically in the SCID-IBD mouse model.

### Example 2

### Preparation of a tablet comprising Compound A and pimecrolimus

Per tablet having a total weight of ca. 750 mg of a solid dispersion of 30 mg of pimecrolimus and a solid dispersion of 1.00 mg Compound A with 160 mg of hydroxypropylmethylcellulose (3 cps) as a carrier are mixed with 8.90 g of lactose (200 mesh), 0.10 mg of butylated hydroxytoluene, 381.25 mg of lactose spray dried, 150.00 mg of crospovidone, 11.25 mg of silicium dioxide colloidal (Aerosil 200®) and 7.50 mg of magnesium stearate are mixed, the mixture obtained is subjected to dry granulation and the granulated mixture obtained is compressed into tablets of 750 mg (+/- 10%) of total weight
A tablet for oral use is obtained.

## Claims

1. The use of a combination of rapamycin or a rapamycin derivative and a compound of formula 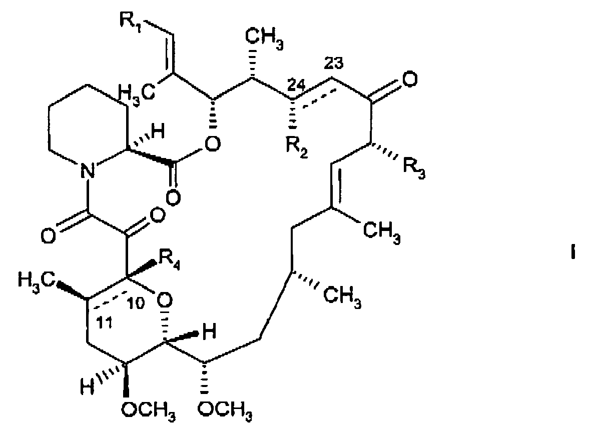 wherein either
R₁ is a group (a) of formula 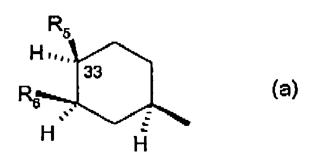 wherein
R₅ is chloro, bromo, iodo or azido,
R₆ is hydroxy or methoxy, and
R₄ is hydroxy, and there is a single bond in 10,11 position; or R₄ is absent, and there is a double bond in 10,11 position,
or
R₁ is a group (b) or (c) of formula 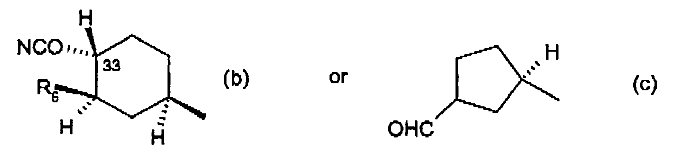 wherein
R₆ is as defined above, and
R₄ is hydroxy and there is a single bond in 10, 11 position,
R₂ is oxo and there is a single bond in 23,24 position; or,
R₂ is optionally protected hydroxy and there is a single or double bond in 23,24 position; or
R₂ is absent and there is a double bond in 23,24 position, and
R₃ is methyl, ethyl, propyl or allyl,
for the manufacture of a medicament for the treatment of inflammatory bowel disease and diseases associated therewith..

2. The use of claim 1, wherein a compound of formula I is a compound of formula

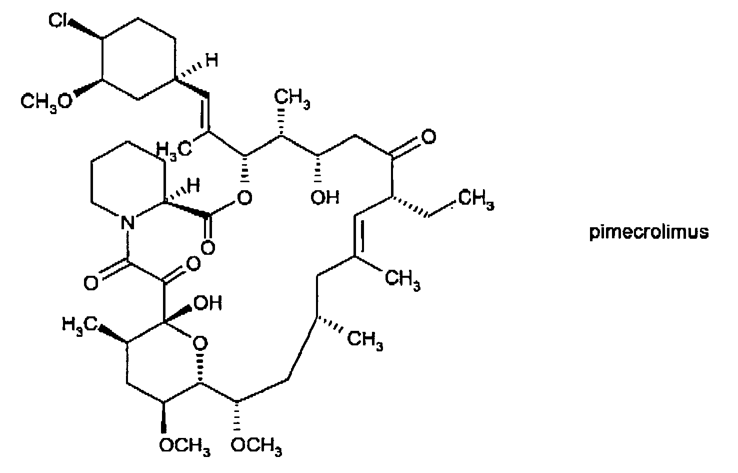

3. The use of any one of claims 1 or 2, wherein the rapamycin derivative is a compound of formula 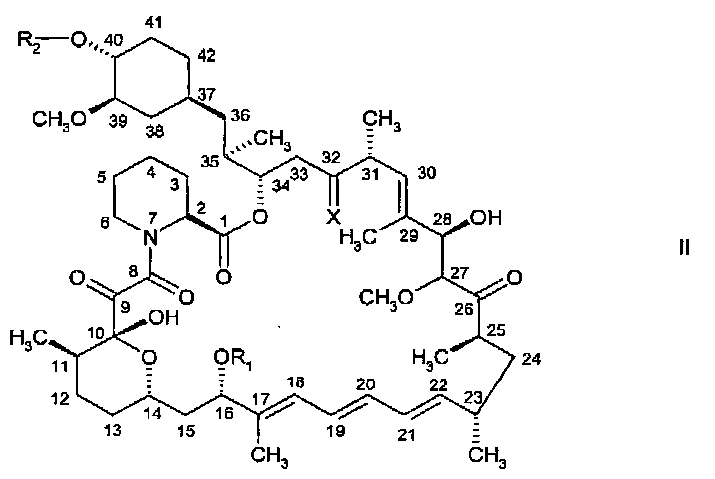 wherein
R₁ is methyl or (C₃₋₆)alkynyl,
R₂ is H, -CH₂-CH₂-OH, 3-hydroxy-2-(hydroxymethyl)-2-methyl-propanoyl or tetrazolyl,
X is =O, (H,H) or (H,OH),
provided that R₂ is other than H when X is =O and R₁ is CH₃,
or a prodrug thereof when R₂ is -CH₂-QH₂-OH.

4. The use of any one of claims 1 or 3, wherein the rapamycin derivative is 40-O-(2-hydroxyethyl)-rapamycin.

5. The use of any one of claims 1 or 4, wherein a compound of formula I is pimecrolimus.

## Patentansprüche

1. Verwendung einer Kombination von Rapamycin oder einem Rapamycinderivat und einer Verbindung der folgenden Formel 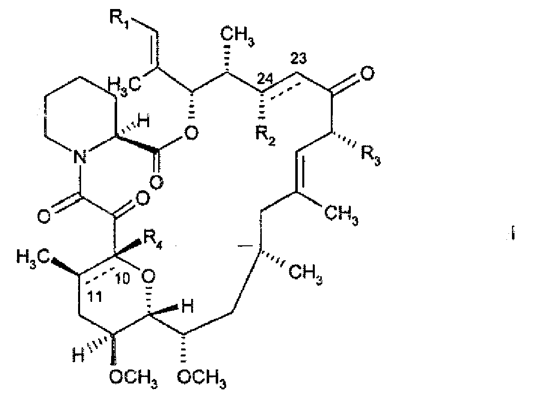 worin entweder
R₁ eine Gruppe (a) der folgenden Formel ist 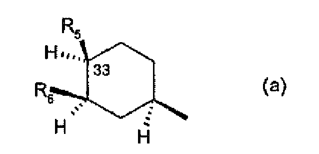 worin
R₅ für Chlor, Brom, Iod oder Azido steht,
R₆ für Hydroxy oder Methoxy steht, und
R₄ für Hydroxy steht und sich in der Position 10,11 eine Einzelbindung befindet, oder R₄ fehlt und sich in der Position 10,11 eine Doppelbindung befindet,
oder
R₁ für eine Gruppe (b) oder (c) der folgenden Formeln steht 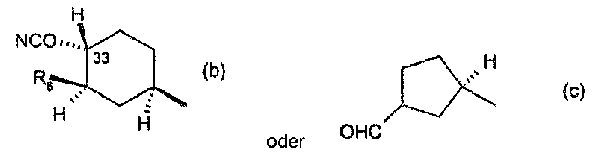 worin
R₆ wie oben definiert ist, und
R₄ für Hydroxy steht und sich in der Position 10,11 eine Einzelbindung befindet,
R₂ für Oxo steht und sich in der Position 23,24 eine Einzelbindung befindet, oder
R₂ optional geschütztes Hydroxy ist und sich in der Position 23,24 eine Einzelbindung oder eine Doppelbindung befindet, oder
R₂ fehlt und sich in der Position 23,24 eine Doppelbindung befindet, und
R₃ für Methyl, Ethyl, Propyl oder Allyl steht,
zur Herstellung eines Arzneimittels für die Behandlung inflammatorischer Darmkrankheiten und damit assoziierter Krankheiten.

2. Verwendung nach Anspruch 1, worin eine Verbindung der Formel I eine Verbindung der folgenden Formel ist

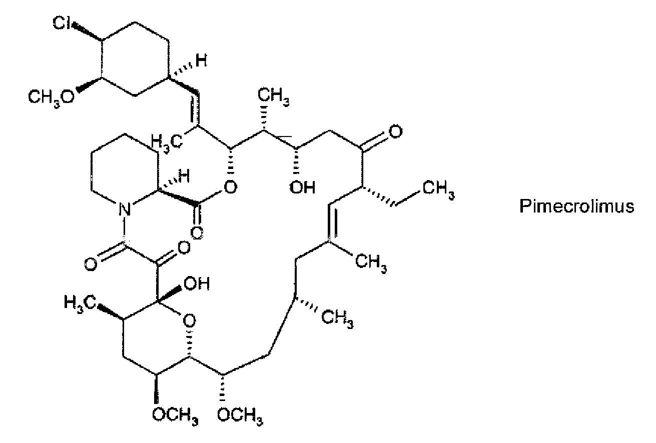

3. Verwendung nach einem der Ansprüche 1 oder 2, worin das Rapamycinderivat eine Verbindung der folgenden Formel ist 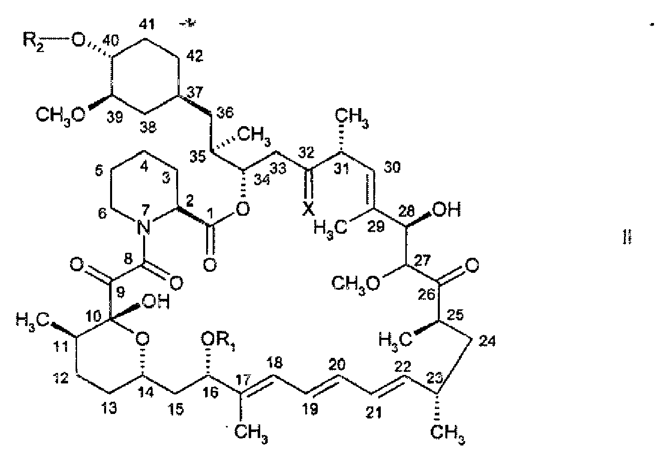 worin
R₁ für Methyl oder (C₃-C₆)-Alkinyl steht,
R₂ für H, -CH₂-CH₂-OH, 3-Hydroxy-2-(hydroxymethyl)-2-methylpropanoyl oder Tetrazolyl steht,
X für =O, (H,H) oder (H,QH) steht,
mit der Maßgabe, dass R₂ etwas anderes als H ist, wenn X für =O steht und R₁ für CH₃ steht,
oder eines Prodrugs hiervon, wenn R₂ für-CH₂-CH₂OH steht.

4. Verwendung nach einem der Ansprüche 1 oder 3, worin das Rapamycinderivat 40-O-(2-Hydroxyethyl)-rapamycin ist.

5. Verwendung nach einem der Ansprüche 1 oder 4, worin eine Verbindung der Formel I Pimecrolimus ist.

## Revendications

1. Utilisation d'une combinaison de rapamycine ou d'un dérivé de rapamycine et d'un composé de formule 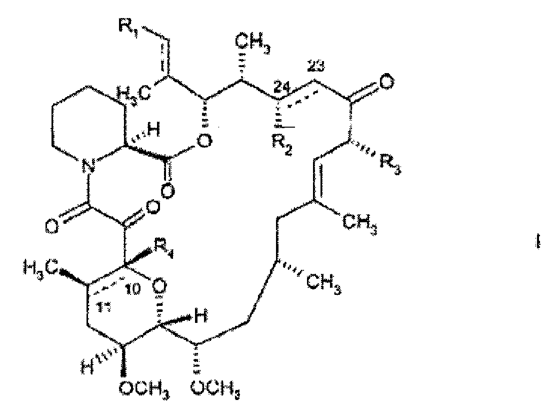 dans laquelle soit
R₁ est un groupe (a) de formule 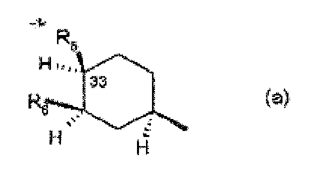
dans laquelle
R₅ est un chloro, un bromo, un iodo ou un azido,
R₆ est un hydroxy ou un méthoxy, et
R₄ est un hydroxy, et il y a une liaison simple en position 10, 11 ; ou R₄ est absent, et il y a une double liaison en position 10, 11
soit
R₁ est un groupe (b) ou (c) de formule 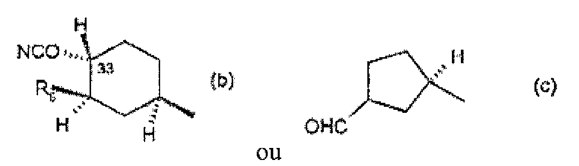
dans lesquelles
R₆ est tel que défini ci-dessus, et
R₄ est un hydroxy et il y a une liaison simple en position 10, 11,
R₂ est un oxo et il y a une liaison simple en position 23, 24 ; ou,
R₂ est éventuellement un hydroxy protégé et il y a une liaison simple ou une double liaison en position 23, 24 ; ou
R₂ est absent et il y a une double liaison en position 23, 24, et
R₃ est un méthyle, un éthyle, un propyle ou un allyle,
pour la fabrication d'un médicament pour le traitement des maladies inflammatoires de l'intestin et des maladies qui y sont associées.

2. Utilisation selon la revendication 1, dans laquelle un composé de formule I est un composé de formule

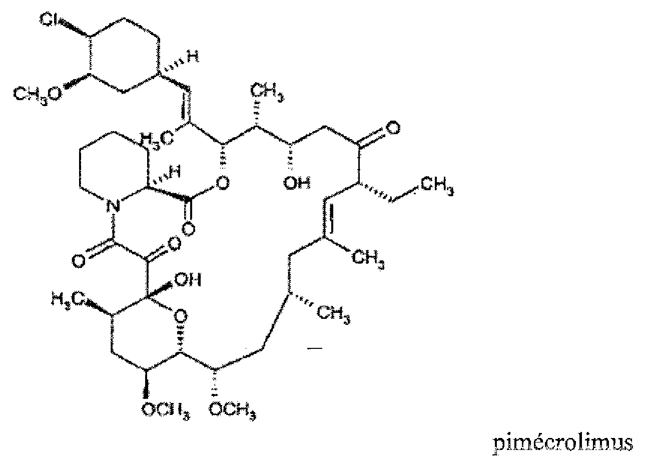

3. Utilisation selon l'une quelconque des revendications 1 ou 2, dans laquelle le dérivé de rapamycine est un composé de formule 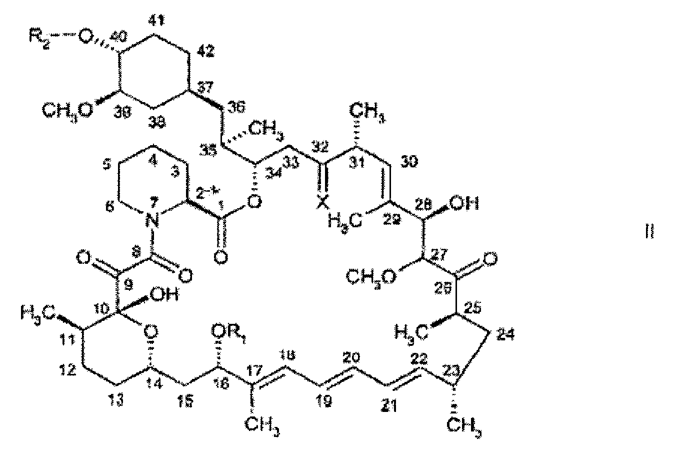 dans laquelle
R₁ est le méthyle ou un alcynyle en (C₃₋₆),
R₂ est H, -CH₂-CH₂-OH, 3-hydroxy-2-(hydroxyméthyl)-2-méthyl-propanoyle ou tétrazolyle,
X est =O, (H,H) or (H,OH),
à condition que R₂ soit autre que H lorsque X est =O et R₁ est CH₃,
ou un promédicament de celui-ci lorsque R₂ est -CH₂-CH₂-OH.

4. Utilisation selon l'une quelconque des revendications 1 ou 3, dans laquelle le dérivé de rapamycine est de la 40-O-(2-hydroxyéthyl)-rapamycine.

5. Utilisation selon l'une quelconque des-revendications 1 ou 4, dans laquelle un composé de formole I est le pimécrolimus.
